# EUROPEAN PATENT APPLICATION

(11) **EP 0 620 280 A1**
(43) Date of publication of application: **19.10.1994**
(21) Application number: 94302414.1
(22) Date of filing: 05.04.1994
(51) Int. Cl.: C12N 15/70, C12N 15/76, C12N 1/21

(54) **Cosmid vectors for streptomycetes genomic DNA cloning**

(30) Priority: 16.04.1993 US 48719
(71) Applicant: PFIZER INC., New York, N.Y. 10017 (US)
(72) Inventor: Denoya, Claudio D., Groton, Connecticut 06340 (US)
(74) Representative: Moore, James William, Dr.

(57) **Abstract**

Novel cosmid cloning vectors are disclosed. Such vectors facilitate many steps involved in cloning chromosomal DNA restriction fragments from streptomycetes. The cosmids have Adenine and Thymine-rich recognition sequences for several restriction enzymes flanking cloning sites. Also disclosed are host cells containing the novel cosmid cloning vectors.

SEQUENCE LISTING

(1) GENERAL INFORMATION
(i) APPLICANT
(A) NAME : Pfizer Inc.
(B) STREET : 235 East 42nd Street, 20th Floor
(C) CITY : New York
(D) STATE : New York
(E) COUNTRY : U.S.A.
(F) POSTAL CODE : 10017-5755

(ii) TITLE OF INVENTION : Cosmid Vectors for Streptomycetes
Genomic DNA Cloning

(iii) NUMBER OF SEQUENCES : 5

(iv) COMPUTER READABLE FORM:
(A) MEDIUM TYPE : Floppy Disk
(B) COMPUTER : IBM PC Compatible
(C) OPERATING SYSTEM : PC-DOS/MS-DOS
(D) SOFTWARE : PatentIn Release #1.0, Version #1.25

(2) INFORMATION FOR SEQ ID NO:1:
(i) SEQUENCE CHARACTERISTICS
(A) LENGTH : 70 amino acids
(B) TYPE : amino acid
(C) STRANDEDNESS : single
(D) TOPOLOGY : linear

(ii) MOLECULE TYPE : DNA (genomic)

(iii) HYPOTHETICAL : NO

(xi) SEQUENCE DESCRIPTION: SEQ ID NO:1:
Ala Gly Ala Ala Thr Thr Cys Gly Cys Gly Gly Cys Cys Gly Cys Ala
1               5                   10                  15
Gly Thr Thr Ala Ala Cys Cys Cys Thr Cys Ala Cys Thr Ala Ala Ala
            20                  25                  30
Thr Gly Ala Thr Cys Cys Cys Thr Ala Thr Ala Gly Thr Gly Ala Gly
        35                  40                  45
Cys Cys Gly Thr Ala Thr Thr Ala Thr Gly Cys Gly Gly Cys Cys Gly
    50                  55                  60
Gly Ala Ala Thr Thr Cys
65                  70
(2) INFORMATION FOR SEQ ID NO:2:
(i) SEQUENCE CHARACTERISTICS:
(A) LENGTH: 12 amino acids
(B) TYPE: amino acid
(C) STRANDEDNESS: single
(D) TOPOLOGY: linear
(ii) MOLECULE TYPE: DNA (genomic)
(xi) SEQUENCE DESCRIPTION: SEQ ID NO:2:
Cys Gly Ala Thr Ala Ala Thr Ala Thr Thr Ala Thr
1               5                   10
(2) INFORMATION FOR SEQ ID NO:3:
(i) SEQUENCE CHARACTERISTICS:
(A) LENGTH: 52 amino acids
(B) TYPE: amino acid
(C) STRANDEDNESS: single
(D) TOPOLOGY: linear
(ii) MOLECULE TYPE: DNA (genomic)

(xi) SEQUENCE DESCRIPTION: SEQ ID NO:3:

Gly Ala Thr Cys Cys Thr Cys Thr Ala Gly Ala Thr Thr Ala Ala
Thr
1               5                   10                  15

Thr Thr Ala Ala Ala Cys Thr Cys Gly Ala Gly Ala Thr Cys Thr
Cys
            20                  25                  30

Gly Ala Gly Thr Thr Thr Ala Ala Ala Thr Thr Ala Ala Thr Cys
Thr
        35                  40                  45

Ala Gly Ala Gly
    50

(2) INFORMATION FOR SEQ ID NO:4:

(i) SEQUENCE CHARACTERISTICS:
(A) LENGTH: 27 amino acids
(B) TYPE: amino acid
(C) STRANDEDNESS: single
(D) TOPOLOGY: linear

(ii) MOLECULE TYPE: DNA (genomic)

(xi) SEQUENCE DESCRIPTION: SEQ ID NO:4:

Gly Ala Thr Cys Cys Thr Thr Thr Ala Ala Ala Gly Ala Thr Cys
Thr
1               5                   10                  15

Cys Gly Ala Gly Thr Thr Thr Ala Ala Ala Gly
            20                  25

(2) INFORMATION FOR SEQ ID NO:5:

(i) SEQUENCE CHARACTERISTICS:
(A) LENGTH: 37 amino acids
(B) TYPE: amino acid
(C) STRANDEDNESS: single
(D) TOPOLOGY: linear

(ii) MOLECULE TYPE: DNA (genomic)

(xi) SEQUENCE DESCRIPTION: SEQ ID NO:5:
Gly Ala Thr Cys Cys Ala Thr Thr Ala Ala Thr Thr Thr Ala Ala
Ala
1 5 10 15
Gly Ala Thr Cys Thr Cys Gly Ala Gly Thr Thr Thr Ala Ala Ala
Thr
20 25 30
Thr Ala Ala Thr Gly
        35

## Description

### Background of the Invention

The present invention relates to a set of novel cosmid cloning vectors that facilitate gene cloning in a wide variety of streptomycetes, including the avermectin-producing microorganism Streptomyces avermitilis.

The application of recombinant DNA techniques in antibiotic-producing microorganisms constitutes an increasingly important area of biotechnological research. Streptomycetes are gram-positive microorganisms that are well known for their ability to produce a variety of commercially useful therapeutic substances. Over 60% of known naturally-occurring antibiotics come from the genus Streptomyces. The development of gene cloning technology in streptomycetes is opening new possibilities in the industrial utilization of streptomycetes for the production of useful compounds. Recombinant DNA techniques are presently applied to improve screening strategies and titres of fresh isolates; to generate novel structures which are not made in naturally-occurring isolates; to clone and express antibiotic biosynthetic genes in heterologous hosts; and to program, at the genetic level, new strains having the ability to perform novel or improved specific tasks not found in the original parental isolates.

Cosmid vectors are modified plasmids that carry at least one copy of the DNA sequences (cos sequences) required for packaging DNA into bacteriophage lambda particles. Cosmids carry an origin of replication (usually the ColE1 origin) and a drug resistance marker (usually ampicillin-resistance), and they can be introduced into E. coli by standard transformation procedures and propagated as plasmids. Cosmid vectors are valuable instruments for cloning large regions of chromosomal DNA because of their ability to carry fragments of genomic DNA ranging in size from about 35 to about 45 kilobases (kb).

Streptomyces DNA has an extremely high guanosine and cytosine content (about 70%). In addition, streptomycetes have relatively large genomes (approximately 10⁷ base pairs). Thus, their DNA content is about three times that of Escherichia coli. This genome complexity makes the cloning of large fragments particularly useful when constructing streptomycetes DNA libraries. Assuming random DNA cleavage and optimal representation of the chromosome in the clone library, the number of clones to be screened is directly related to genome size and inversely related to the average size of the DNA inserts. Therefore, it is advantageous to clone large pieces of DNA in order to reduce the number of clones to be screened for a particular gene. Reducing the number of- clones also reduces the number of analyses and manipulations required to establish a restriction map of a large chromosomal region by identifying contiguous or overlapping clones.

The present invention provides novel cosmid cloning vectors that facilitate both the cloning of large chromosomal DNA fragments (i.e., a large fragment of chromosomal DNA carrying a cluster of antibiotic biosynthetic genes) and the recovery of these cloned fragments as single pieces from the vector.

### Summary of the Invention

The present invention describes a novel set of cosmids having adenine- plus thymine-rich (hereinafter "[A+T]-rich") recognition sequences for several restriction enzymes flanking the cloning site(s). Since [A+T]-rich recognition sequences are infrequently represented in the naturally occurring streptomycetes genome, the present invention increases the chances of finding at least one of these enzymes that will allow any particular guanine- plus cytosine-rich (hereinafter "[G+C-rich]") genomic DNA insert to be removed as a single restriction fragment. This feature is useful for the in vitro recovery and manipulation of large genes or cluster of genes, such as antibiotic biosynthetic gene clusters. In addition, these vectors may contain T3 and T7 RNA polymerase promoters flanking the cloning site, allowing synthesis of RNA transcripts for rapid directional genomic walking, and restriction mapping. The present invention also relates to an E. coli - Streptomyces shuttle cosmid vector useful for the construction of Streptomyces DNA libraries in E. coli which can then be transferred into Streptomyces. These vectors facilitate genetic engineering of Streptomyces species and other relevant microorganisms with high [G+C-rich] genomic content. This feature is useful for the in vitro reconstruction of large chromosomal regions (such as those carrying clustered genes, particularly antibiotic biosynthetic genes).

Broadly, the invention relates to cosmid cloning vectors comprising: an E. coli origin of replication; at least one DNA segment that - conveys resistance to an antibiotic when transformed into a sensitive E. coli host cell; at least one cos sequence from bacteriophage; at least one cloning site flanked by [Adenine+Thymine]-rich recognition sequences for restriction enzymes; and at least two different RNA polymerase promoters flanking the polylinker region.

In preferred embodiments, the present invention relates to a cosmid cloning vector comprising: (a) a PstI/ClaI DNA restriction fragment of about 4.2 kilobases, said restriction fragment comprising a ColEI origin of replication and at least one bacteriophage lambda cos site; (b) a PstI/ClaI DNA fragment of about 0.8 kilobases, said DNA fragment comprising at least one BamHI cloning site flanked by at least two different bacteriophage promoter sequences and said BamHI cloning site further flanked by at least two restriction sites (SEQ ID NO:1); and (c) a DNA segment containing at least one antibiotic marker that confers resistance when transformed into a sensitive E. coli host cell. More preferably the cosmid cloning vector further comprises: (d) an SsPI linker inserted at the ClaI site (SEQ ID NO:2); and (e) at least three additional cloning sites designated as EcoRI, Noti and BamHI. Especially preferred is a cosmid cloning vector wherein the 4.2kb PstI/ClaI DNA restriction fragment is derived from plasmid pCD184; the 0.8 kb PstI/ClaI DNA fragment is derived from pWE15 (SEQ ID NO:1), the BamHI cloning site is flanked by at least promoter sequences from bacteriophages T3 and T7 and the BamHI cloning site is further flanked by at least two restriction sites for enzymes NotI and EcoRI (SEQ ID NO:1); and an SspI linker is inserted at the ClaI site described for plasmids pCD184 and pCD193 (SEQ ID NO:2). The cosmid cloning vector may be a plasmid, such as the plasmids designated as pCD188, pCD382, pCD383, pCD397, and pCD418. The present invention also relates to an E. coli host cell comprising plasmids such as pCD188, pCD382, pCD383, pCD397, and pCD418. The cosmid cloning vector may comprise all of the structural characteristics of plasmid pCD188; and it may further comprise: an additional XhoI cloning site located adjacent the BamHI cloning site, or additional XhoI and XbaI cloning sites located adjacent the BamHI cloning site; or a multiple cloning site with the following configuration: EcoRI/NotI/BamHI/DraI/BglII/XhoI/DraI/BamHI/NotI/EcoRI, or a multiple cloning site with the following configuration: EcoRI/NotI/BamHI/AseI/DraI/BglII/XhoI/DraI/AseI/BamHI/NotI / EcoRi. The present invention also relates to an E. coli host cell comprising the above-referenced cosmid cloning vectors.

The present invention is also directed to a bi-functional, low copy number, shuttle cosmid cloning vector which comprises [Adenine+Thymine]-rich recognition sequences for restriction enzymes flanking the cloning site. In preferred embodiments, the DNA cosmid shuttle cloning vector comprises: (a) an E. coli origin of replication comprising a BamHI restriction fragment of about 5 kilobases; (b) a DNA segment comprising at least one antibiotic marker that confers resistance when transformed into an E. coli host cell; (c) a BglII restriction fragment of about 25 kilobases comprising a streptomycetes origin of replication; (d) a DNA segment comprising at least one antibiotic marker that confers resistance when transformed into a sensitive Streptomyces host cell; (e) a BamHI cloning site; and (f) two ClaI sites flanking the BamHI cloning site. The BamHI restriction fragment may be that of plasmid pCD188, and the BglII restriction fragment may be that of plasmid pIJ940. The vector may be capable of replicating efficiently in a wide variety of streptomycetes and/or E. coli hosts, and it may be a plasmid such as the plasmid designated pCD425. The present invention also relates to a streptomycetes or E. coli host cell comprising a shuttle vector, and the host cell may be selected from Streptomyces avermitilis; Streptomyces coelicolor; Streptomyces hygroscopicus; and Streptomyces lividans, and the cloning shuttle vector may be plasmid pCD425.

These and other objects and advantages of the present invention will become apparent from the following description of the accompanying figures which disclose several embodiments of the invention. It is to be understood that the figures are to be used for the purposes of illustration only and not as a definition of the invention.

### Brief Description of the Figures

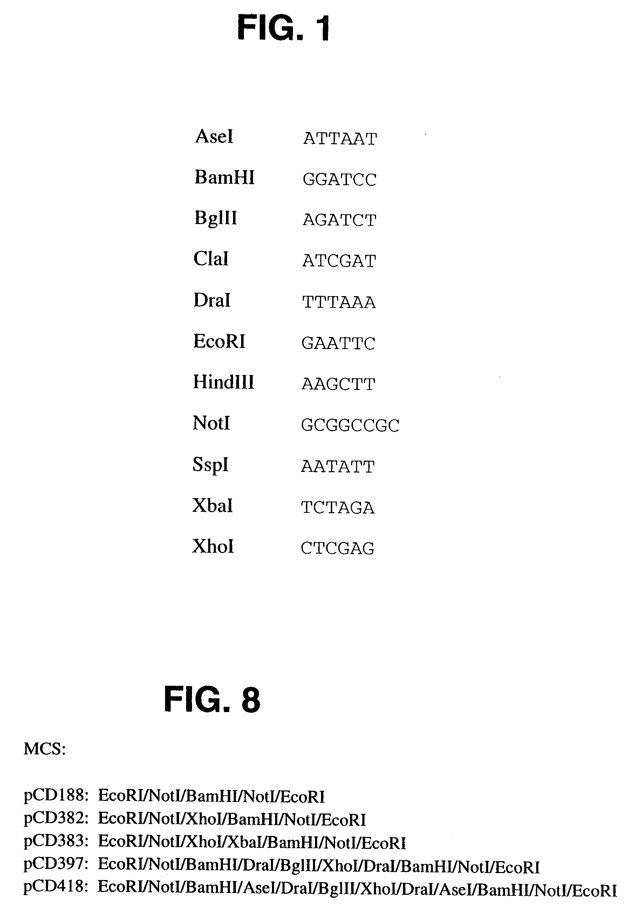

FIG. 1 shows the DNA recognition sequence for restriction enzymes used in the present invention.

FIG. 2 shows the restriction map for plasmid pPFZ543.

FIG. 3 shows the restriction map for plasmid pWE15.

FIG. 4 shows the restriction map for plasmid pCD184.

FIG. 5 shows the restriction map for plasmid pCD193.

FIG. 6 shows the construction sequence for plasmid pCD188.

FIG. 7 shows the restriction map for plasmid pCD188.

FIG. 8 shows the restriction recognition sites present in the Multiple Cloning site (MCS) of plasmids pCD188, pCD382, pCD383, pCD397 and pCD418.

FIG. 9 shows the restriction map for plasmid pCD425.

### Detailed Description of the Invention

Technical terms used throughout this application are well known to those skilled in the art of molecular genetics. Definition of those terms are found in many textbooks dedicated to the molecular biology field, such as "Genes", Second Edition, by Dr. Benjamin Lewin, 1985, John Wiley & Sons, Inc. New York. Terms frequently utilized in this invention are defined below (all of the documents cited herein, including the foregoing, are incorporated in this application in their entireties for all purposes):
Antibiotic: A chemical agent that inhibits growth of bacterial cells. It may be used to select recombinant bacterial cells.
Antibiotic Resistance Gene: A DNA sequence that conveys resistance to an antibiotic when introduced into a host cell that is naturally sensitive to that particular antibiotic. It is also known as an antibiotic marker.
Bacteriophage: Virus that infect bacteria.
cRNA: A single-stranded RNA complementary to a DNA, synthesized from the DNA by in vitro transcription.
Clone: A large number of cells or molecules identical to a single ancestor.
Cloning Vector: Any plasmid into which a foreign DNA may be inserted to be cloned. It carries foreign DNA into a host cell upon transformation.
Cohesive End Sequence (Cos): A DNA sequence derived from bacteriophage lambda allowing in vitro packaging.
Cosmid: A plasmid into which bacteriophage lambda cos sites have been inserted. The plasmid DNA (carrying foreign DNA inserts) can be packaged in vitro in the phage coat.
DNA Ligation: The formation of a chemical bond to link two fragments of DNA.
Hybridization: A technique used to identify bacterial colonies carrying chimeric vectors whose inserted DNA is similar with some particular sequence.
kb: An abbreviation for 1,000 base pairs of DNA or RNA.
Linker: A short synthetic duplex oligodeoxynucleotide containing the target site for one or more restriction enzymes. It is added to a vector to create a novel polylinker or multiple cloning site (MCS).
Origin of Replication: A DNA sequence at which replication of the plasmid vector is initiated.
Plasmid: Autonomous self-replicating extrachromosomal circular DNA.
Plasmid Copy Number: Number of plasmid molecules maintained in bacteria for every host chromosome.
Promoter: A Region of DNA which is important for defining the initiation of transcription of a particular DNA sequence into RNA.
Replicon: A unit of genome in which DNA is replicated. It contains an origin of replication.
Restriction Enzyme: An enzymatic protein that recognizes a specific short sequence of DNA and cleaves it.
Restriction Recognition Sequence: A DNA sequence specifically recognized by a particular restriction enzyme. It is also known as a target site.
Shuttle Vector: A bifunctional cloning vector capable of replicating in one or more alternative hosts (e.g., E. coli and Streptomyces).
Transcription: The synthesis of RNA on a DNA template.
Transformation of Bacterial Cells: The acquisition of new genetic markers by the incorporation of added DNA.

The novel cosmid vectors of the present invention facilitate the isolation of sequentially overlapping cosmid clones using strategies known as "chromosomal walking". In preferred embodiments, these cosmid vectors contain bacteriophage promoter sequences (for instance, T3 and T7) on either side of the cloning site. By using the cosmid DNA containing a genomic insert as a template for either T3 or T7 polymerase, directional "walking" probes can be synthesized in vitro, and used to screen genomic cosmid libraries.

In addition, some of the cosmid vectors disclosed herein, such as pCD382, pCD383, pCD397, and pCD418, have an XhoI cloning site. The availability of XhoI as a cloning site is described by Zabarovsky and Allikmets, Gene, 42:119, 1986. The cloning method of the present invention utilizes the unique specificity with which partially filled-in XhoI cohesive termini present at both ends of a XhoI-linearized vector can be combined with partially filled-in Sau3AI digested genomic DNA. The only ligation products possible are single copies of genomic inserts, since the partial fill-in prevents self-ligation reactions of vector and genomic fragments. This method is also quick, requires small amounts of starting material, and makes genomic fractionation unnecessary.

In preferred embodiments, the present invention relates to a novel cosmid vector that is bi-functional, i.e., capable of replication in both E. coli and in streptomycetes. This vector can therefore shuttle between Streptomyces and E. coli. Gene cloning can be carried out in E. coli, and the selected recombinant vector, carrying a large insert (such as a large chromosomal DNA fragment carrying the genes for a complex biosynthetic pathway), can then be transformed into streptomycetes for functional analysis.

The shuttle vector of the present invention may carry the SCP2* Streptomyces origin of replication that controls for a low copy number in Streptomyces. Such an ability to control for low copy number in Streptomyces is an advantageous characteristic for vectors utilized in cloning and complementation studies in many Streptomyces species. SCP2* is a fertility factor originally found in Streptomyces coelicolor with a molecular size of 31.4 kb and exists in one to two copies per chromosome (Bibb, M. J. and Hopwood, D. A., J. Gen. Microbiol., 126, pp 427-442, 1981). SCP2* has a broad host range. The SCP2* replicon carried in the cosmid shuttle vector may be derived from the low copy number vector pIJ940 (Lydiate et al., Gene, 35, pp. 223-235, 1985). This vector carries the thiostrepton-resistant (tsr) marker from Streptomyces azureus, which confers resistance to the antibiotic thiostrepton by a specific ribosomal RNA methylase. Vectors with low or single copy number per genome are preferred for most physiological studies due to their ability to stably maintain large DNA fragments (up to 40 kb) and the absence of possible gene dosage effects.

The present invention also relates to novel cosmid vectors designated pCD188, 382, 383, 397, and 418, and a bi-functional (shuttle) vector (pCD425) for cloning and analysis of Streptomyces DNA and other [G+C]-rich genomic DNAs.

The cosmid vector pCD188 of the present invention comprises:
a. an approximately 4.2 kb PstI/ClaI DNA fragment from pCD184 containing a ColE1 origin of replication and a single bacteriophage lambda cos site;
b. an approximately 0.8 kb PstI/ClaI DNA fragment from pWE15 containing: a BamHI cloning site flanked by bacteriophage promoter sequences (T3 and T7) on either side of the cloning site (in addition it contains two restriction sites flanking the mentioned cloning site for each of the following restriction enzymes: NotI, and EcoRI (SEQ ID NO:1));
c. an SspI linker inserted at the single ClaI site (thus, cosmid vector pCD188 has two SspI sites flanking the single BamHI (SEQ ID NO:2)). The recognition sequence for the SspI restriction enzyme (AATATT) is 100% composed of Adenine and Thymine residues, which makes this sequence very rare in [G+C]-rich genomes such as streptomycetes. Therefore, by using SspI there is a good chance of removing a streptomycetes cloned DNA insert as a single restriction fragment); and
d. a DNA sequence that conveys resistance to the antibiotic ampicillin when transformed into a sensitive E. coli host cell.

The cosmid vectors pCD382, pCD383, pCD397, and pCD418 were constructed by inserting particular polylinkers in the single BamHI site of cosmid vector pCD188. As a result of these constructions, several additional useful characteristics were obtained including the following:
a. An additional XhoI cloning site next to the single BamHI site in pCD382, which allows implementation of the "partially filled-in XhoI" strategy for simplified genomic cloning procedures as described above (see also Zabarovsky and Allikmets, 1986);
b. Additional XhoI and XbaI cloning sites next to the single BamHI in pCD383 (SEQ ID NO:3);
c. A multiple cloning site in pCD397 with the following configuration: EcoRI/NotI/BamHI/DraI/BglII/XhoI/ DraI/BamHI/NotI/EcoRI (SEQ ID NO:4) (since the recognition sequence for DraI is exclusively composed of Adenine and Thymine residues (TTTAAA), and there are 2 DraI sites flanking the BglII and XhoI cloning sites, it is possible to recover with a great chance any [G+C]-rich DNA insert cloned at either the BglII or the XhoI sites as a single fragment);
d. A multiple cloning site in pCD418 with the following configuration: EcoRI/NotI/BamHI/AseI/DraI/BglII/ XhoI/DraI/AseI/BamHI/NotI/EcoRI (SEQ ID NO:5) (since the recognition sequences for AseI and DraI are exclusively composed of Adenine and Thymine residues (ATTAAT and TTTAAA, respectively), and there are 2 AseI and 2 DraI sites flanking the BglII and XhoI cloning sites, it is possible to recover with a great chance any [G+C]-rich DNA insert cloned at either the BglII or the XhoI sites as a single fragment by digesting the recombinant clone with either AseI or DraI restriction endonucleases).

The cosmid shuttle vector pCD425 of the present invention, comprises:
a. an approximately 5 kb pCD188 moiety containing: a ColEI origin of replication, a single cos site, and an ampicillin resistance marker which confers resistance upon transformation to ampicillin-sensitive E. coli host cells; and
b. an approximately 25 kb pIJ940 moiety containing a SCP2* origin of replication, and two antibiotic resistance markers (thiostrepton and hygromycin) that confer resistance upon transformation to streptomycetes sensitive host cells (see Hopwood et al., 1985, "Genetic Manipulation of Streptomyces-A Laboratory Manual," the John Innes Foundation, U.K.).

The cosmid shuttle vector pCD425 has several advantageous characteristics including the following;
a. It has a single BamHI cloning site;
b. It has 2 ClaI sites flanking both sides of the single BamHI site (the [A+T]-rich ClaI recognition sequence (ATCGAT) is not very frequent in [G+C]-rich streptomycetes genomic DNA, thus, recovery of cloned inserts as large pieces is facilitated);
c. It is a useful cosmid cloning vector for medium size genomic fragments (10-20 kb);
d. It has a T7 bacteriophage promoter at one side of the cloning site allowing probe preparation from one end of the inserted DNA;
e. It has shuttle ability because it carries both E. coli and streptomycetes origins of replication;
f. It has a high copy number in E. coli cells facilitating efficient amplification of cloned DNA; and
g. It presents a low copy number in streptomycetes which makes it an ideal choice for complementation studies in a myriad of mutated and wild type strains.

The following have been deposited under the terms of the Budapest Treaty in the American Type Culture Collection, Rockville, Maryland, a recognized depository affording permanence of the deposits and ready accessibility thereto by the public if a patent is granted on this application. The deposits are available during pendency of this application to one determined by the Commissioner of the United States Patent and Trademark Office to be entitled thereto under 37 CFR 1.14 and 35 USC 122, and in accordance with foreign patent laws, in countries wherein counterparts of this application, or its progeny, are filed. All restrictions on the availability to the public of the microorganisms deposited will be irrevocably removed upon granting of the patent.

| ATCC DEPOSITS IN ACCORDANCE WITH THE PRESENT INVENTION | |
|---|---|
| Identification Reference by Depositor Pfizer, Inc. | ATCC Designation |
| Escherichia coli, FD 29380 | 69427 |
| Escherichia coli, FD 29381 | 69428 |
| Escherichia coli, FD 29382 | 69429 |
| Escherichia coli, FD 29383 | 69430 |
| Escherichia coli, FD 29384 | 69431 |
| Escherichia coli, FD 29392 | 69434 |
| S. lividans TK64, FD 29419 | 69445 |

The following detailed Examples relate to the construction of novel cosmid cloning vectors pCD88, pCD382, pCD383, pCD397, pCD418, and pCD425. These Examples illustrate the present invention, however, it will be understood that the invention is not limited to the specific details of these examples.

### EXAMPLE 1

### Culture of E. coli strain CD155 and Isolation of Cosmid Vector pPFZ543

E. coli strain CD155 is an ampicillin-resistant transformant. It was originally obtained by transformation of competent E. coli K12 DH5-α cells with plasmid pPFZ543 following standard protocols as described by Maniatis et al, Molecular Cloning - a laboratory manual, Cold Spring Harbor Laboratory, 1989. Cosmid vector pPFZ543 is an improved derivative of cosmid vector pPFZ514 lacking a dam methylation site present in the polylinker region of the parent vector. Construction and utilization of cosmid vector pPFZ514 was described by Binnie et al., J. Bacteriol., 1989, 171:887.

A single bacterial colony of E. coli strain CD155 was inoculated into LB medium which contains, per liter of aqueous solution, 10 g bacto tryptone, 5 g bacto-yeast extract, and 10 g NaCl(pH 7.0) with 50 mg of ampicillin following standard microbiological procedures. The culture was incubated at 35°C overnight (12-15 hours).

The following morning, the bacterial cells were harvested by centrifugation at 10,000 rpm for 5 minutes at 4°C. Cosmid vector pPFZ543 was isolated from fresh harvested Escherichia coli CD155 cells using a modification of the method of Birnboim and Doly, Nucleic Acids Res., 1979, 7:1513, as described by Denoya et al., Microbios Lett., 1985, 29:87. The isolated plasmid DNA was then dissolved in DNA buffer (10 mM Tris-HCl, 4 mM NaCl, 0.1 mM EDTA; pH 7.5) to produce a concentration of approximately 1 µg of pPFZ543 per 10 µl of buffer.

### EXAMPLE 2

### Construction of Cosmid Vector pCD184

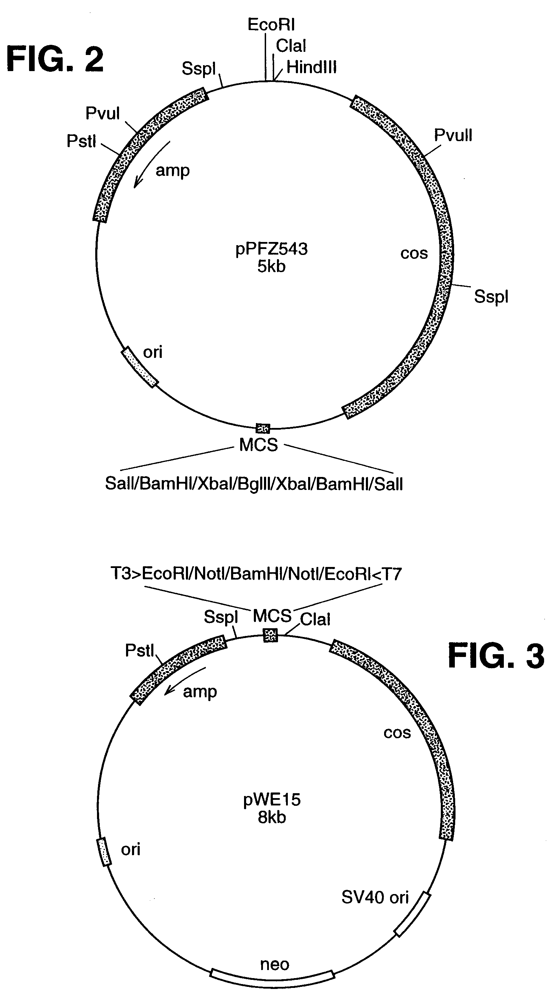

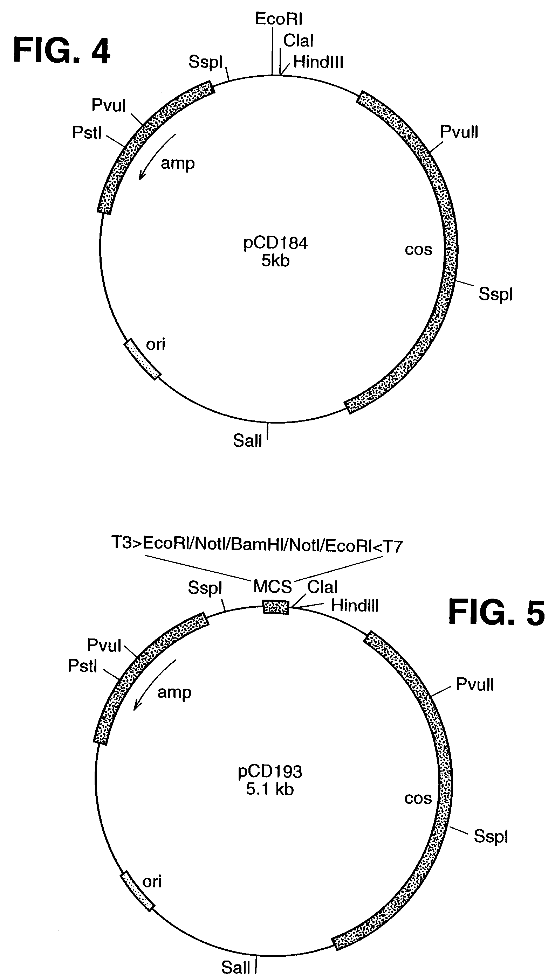

Cosmid vector pCD184 was constructed by removal of the polylinker region of pPFZ543 (see FIG. 2). The "polylinker", "polycloning site", and "multiple cloning site" is a closely arranged series of synthetic cloning sites that consist of sequences recognized by restriction enzymes commonly used in cloning experiments. The polylinker from vector pPFZ543 consists of a tandem array of cleavage sites for: SalI, BamHI, XbaI, BglII, XbaI, BamHI, and SalI. The techniques used for cleaving in vitro plasmid DNA at specific sites, ligating previously cleaved DNA, and the designations of the particular enzymes used are well known to those skilled in molecular biology and are described, for example, in the laboratory manual "Molecular Cloning" by Maniatis et al. (Cold Spring Harbor Laboratory, 1989). The polylinker present in plasmid pPFZ543 was deleted by the following technique: 3 µg of the plasmid pPFZ543 in 30 µl of DNA buffer was incubated with 20 units of the restriction enzyme SalI (all restriction enzymes were purchased from Boehringer Mannheim Biochemicals) in the assay buffer specified by the supplier, at 37°C for 2 hours, in a total reaction volume of 40 µl. Following restriction with SalI, the DNA was extracted twice with an equal volume of phenol-chloroform, twice with an equal volume of ether, and finally the DNA was precipitated by adding 2 volumes of absolute ethanol. Precipitated DNA was recovered by centrifugation at 10,000 x G for 10 minutes and dried under vacuum. The SalI-treated DNA was then dissolved in 12 µl of DNA buffer and incubated overnight (12-15 hours) with 1 unit of ligase (New England BioLabs) under the conditions specified by the supplier at 16°C in a total reaction volume of 20 µl. The reaction was terminated by transferring the assay microtube on ice and 15 µl of the reaction mixture was then used to transform competent E. coli HB101 cells (purchased from Life Technologies, Inc., Gaithersburg, MD) by the method recommended by the suppliers. Many ampicillin-resistant transformants were recovered. It was confirmed that these colonies contained the plasmid pCD184, including a unique SalI restriction site, as follows: One colony, designated as strain CD184, was selected; plasmid DNA was isolated from this strain (by the method already described for pPFZ543 in Example 1), and it was shown that it is cleaved by restriction enzyme SalI, and that it does not contain sensitive sites for the restriction enzymes BamHI, XbaI and BglII (FIG. 4).

### EXAMPLE 3

### Construction of Cosmid Vector pCD193

### A. PstI/ClaI Digestion of Cosmid Vector pCD184 and isolation of the approximately 4.2 kb Restriction Fragment

About 5 µg of plasmid pCD184, 10 units of PstI restriction enzyme and 10 units of ClaI restriction enzyme were incubated in the assay buffer specified by the supplier (Boehringer) at 37°C for 2 hours, in a total reaction volume of 60 µl. The sample was then electrophoresed in a horizontal 1.2% agarose gel in 1X TBE buffer (90 mM Tris-HCl, pH 8.5, 90 mM boric acid, 2.5 mM EDTA) for 1.5 hours at 100 V as described by Maniatis et al. The separated DNA fragments were located in the gel by staining with ethidium bromide and visualizing fluorescent bands with a 365 nm ultraviolet light. The 4.2 kb DNA band was sliced with a razor blade and the DNA recovered from the agarose gel by electroelution for 50 minutes at 80 Volts into a V-shaped well filled with 7.5 M ammonium acetate using an unidirectional electroelutor (International Biotechnology Inc., New Haven, CT). The DNA was then precipitated with ethanol, pelleted and finally redissolved in 10 µl of DNA buffer.

### B. PstI/ClaI digestion of Cosmid Vector pWE15 and isolation of the approximately 0.8 kb Restriction Fragment (SEQ ID NO:1)

pWE15 is a 8.8 kb cosmid vector which contain a BamHI cloning site flanked by T3 and T7 bacteriophage promoters (purchased from Stratagene Cloning Systems, La Jolla, CA) (see FIG. 3). Since pWE15 has 3 PstI/ClaI DNA fragments in the 0.8 kb range, it was first necessary to identify the fragment carrying the cloning site and bacteriophage promoters. This was performed by restriction analysis using five different restriction enzymes: BamHI, ClaI, EcoRI, PstI, and SspI. The identification was done following standard procedures for restriction mapping (see Maniatis et al., 1989) using the pWE15 restriction map obtained from the supplier as a reference. The desired fragment was identified as the smallest of the 3 PstI/ClaI bands, and it was recovered from an agarose gel following a similar procedure as described above, except that the ammonium concentration in the electroelutor well was raised to 10 M to assure efficient recovery of the small fragment. The final DNA pellet was redissolved in 10 µl of DNA buffer.

### C. Ligation to Produce pCD193

About 3 µl of the approximately 4.2 kb PstI/ClaI fragment of plasmid pCD184 (see Example 3A) and about 8 µl of the approximately 0.8 kb PstI/ClaI fragment (SEQ ID NO:1) of plasmid pWE15 (see Example 3B) were incubated overnight (12-15 hours) with 1 unit of ligase (New England BioLabs, Inc., Beverly, MA) under the conditions specified by the supplier at 14°C in a total reaction volume of 20 µl. The following morning, the ligation mixture was used to transform competent E. coli HB101 cells by the method discussed above. A restriction map of pCD193 is presented in FIG.5.

### EXAMPLE 4

### Construction of Cosmid Vector pCD188

A. ClaI Digestion and Dephosphorylation of Cosmid Vector pCD193

Restriction enzyme digestion was performed essentially as described in the above-referenced Examples except that about 5 µg of plasmid pCD193, ClaI restriction enzyme, and appropriate incubation buffer were used. ClaI-linearized pCD193 cosmid vector was then dephosphorylated using calf intestine alkaline phosphatase (CIP) (purchased from Promega Corp., Madison, WI) following the instructions obtained from the supplier. The reaction mixture was incubated for 30 minutes at 37°C, and the DNA was then extracted consecutively with phenol/chloroform and ether, ethanol precipitated, and pelleted by centrifugation. Final pellet was redissolved in 9 µl of DNA buffer.

### B. Phosphorylation of SspI Linker

A SspI linker, 5'-CGATAATATTAT-3' (SEQ ID NO:2), was prepared. A linker DNA pellet (representing 0.89 units of optical absorbance at 260 nm) was resuspended in 30 µl of DNA buffer. The reaction materials consisted of the following: 7 µl of linker DNA, 1 µl of 10X Kinase buffer (700 mM Tris- HCl [pH 7.6], 100 mM MgCl₂, 50 mM DTT), 1 µl of 10 mM ATP, and 1 µl of T4 Polynucleotide Kinase (purchased from Promega Corp). Incubation was for 1 hour at 37°C.

### C. Ligation to Produce pCD188 (SEQ ID NO:1)

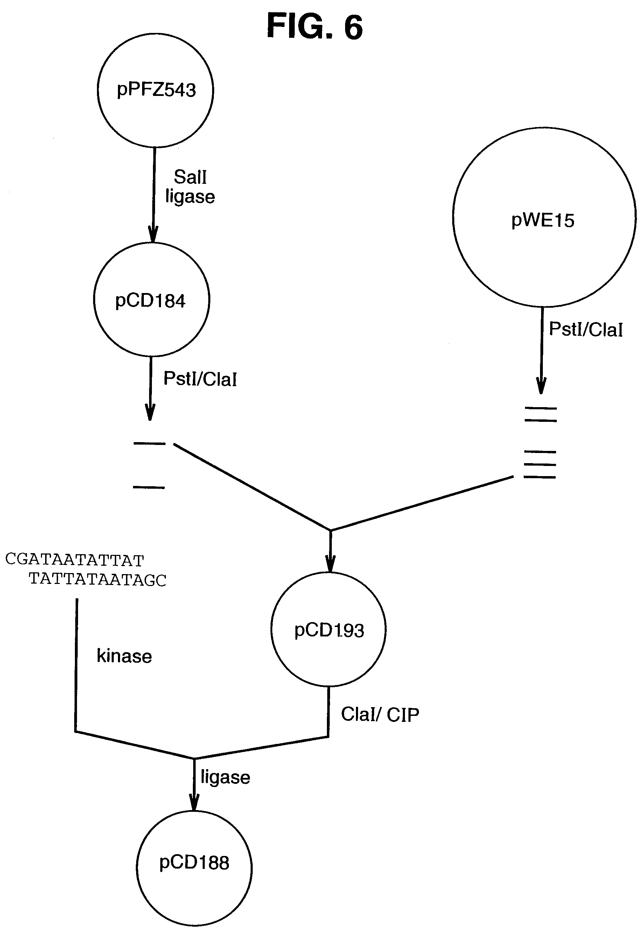

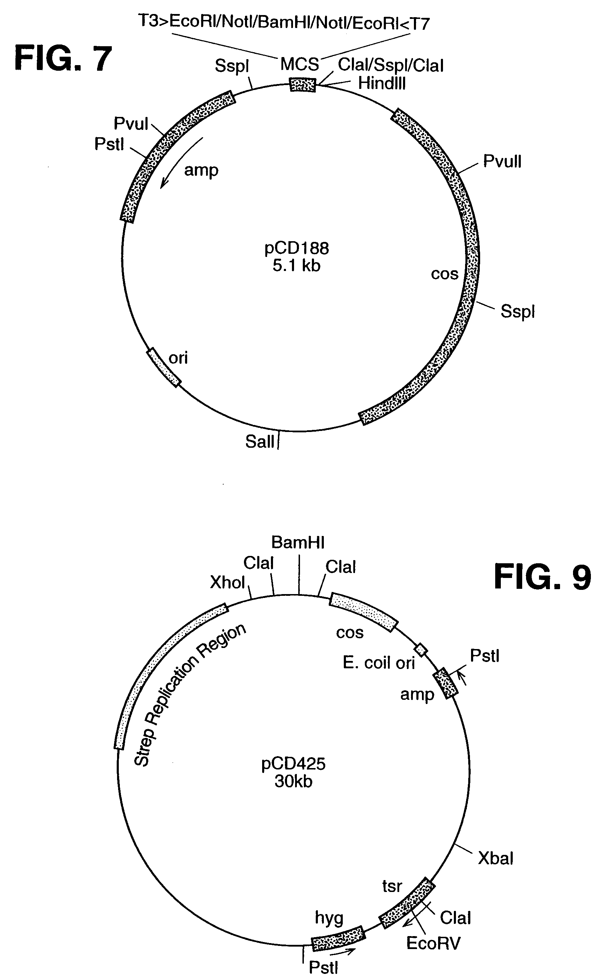

About 9 µl of ClaI-linearized dephosphorylated pCD193 vector (see Example 4A) was added to the linker phosphorylation reaction mixture (see Example 4B). Then, approximately 1.2 µl of 10X Kinase buffer, 1 µl of 10 mM ATP, and 1 µl of ligase (purchased from Collaborative Research, Inc., Bedford, MA) were added sequentially, and the reaction mix was incubated overnight (12-15 hours) at 14°C. Next morning, the ligation reaction was used to transform E. coli HB101 cells by the method discussed above. The selected transformant was designated as strain CD188 (SEQ ID NO:1). The identified transformant was then used for subsequent production and isolation of cosmid vector pCD188. A restriction map of cosmid vector pCD188 is presented in FIG.7. A summary of the construction is presented in FIG. 6.

### EXAMPLE 5

### Construction of Cosmid Vectors pCD382 and pCD383 (SEQ ID NO:3)

### A. BamHI Digestion and Dephosphorylation of Cosmid Vector pCD188 (SEQ ID NO:1)

Restriction endonuclease digestion and dephosphorylation of vector pCD188 were performed essentially as described in Example 4A, except that about 3 µg of vector pCD188, BamHI restriction enzyme and appropriate restriction buffer were used in the restriction endonuclease digestion step.

### B. Phosphorylation of Polylinker

A polylinker, 5'-GATCCTCTAGATTAATTTAAACTCGAGATCTCGAGTTTAAATTAATCTAGAG-3' (SEQ ID NO:3), was prepared. A linker DNA pellet (representing 0.25 units of optical absorbance at 260 nm) was resuspended in 14 µl of DNA buffer. Phosphorylation of this polylinker was performed essentially as described in Example 4B.

### C. Insertion of Polylinker into the BamHI site of pCD188

About half of the amount of phosphorylated polylinker used as described in Example 5B was ligated to about 0.15 µg of BamHI-linearized dephosphorylated pCD188, under conditions similar to those described in the preceding examples. The ligation mixture was used to transform competent E. coli DH5-alpha (DH5-α) cells. Two transformants were selected, and designated as strains CD382 and CD383. Plasmid cosmid vector isolated from strain CD382, pCD382, was found to have the following functional cloning sites: BamHI and XhoI. Plasmid cosmid vector isolated from strain CD383, pCD383, was found to have the following functional cloning sites: BamHI, XbaI, and XhoI (SEQ ID NO:3) (see FIG. 8).

### EXAMPLE 6

### Construction of Cosmid Vectors pCD397 and pCD418

Novel cosmid vectors pCD397 and pCD418 were constructed by insertion of specifically designed polylinkers (linkers #18 and #20, respectively) into the BamHI cloning site of vector pCD188, using technology similar to that described in the preceding example. Two newly designed polylinkers were prepared: linker #18, 5'-GATCCTTTAAAGATCTCGAGTTTAAAG(SEQ ID NO:4)-3', and linker #20, 5'-GATCCATTAATTTAAAGATCTCGAGTTTAAATTAATG(SEQ ID NO:5)-3'. Plasmid cosmid vector isolated from E. coli DH5-alpha (DH5-α) strain CD397, pCD397, was found to have the following functional cloning sites: BamHI, DraI, BglII, and XhoI. Plasmid cosmid vector isolated from E. coli DH5-alpha (DH5-α) strain CD418, pCD418, was found to have the following functional cloning sites: BamHI, AseI, DraI, BglII, and XhoI (see FIG. 8).

### EXAMPLE 7

### Construction of Cosmid Shuttle Vectors pCD425

A. BamHI Digestion and Dephosphorylation of Cosmid Vector pCD188

Restriction endonuclease digestion and dephosphorylation of vector pCD188 were performed essentially as described in Example 4A, except that about 3 µg of vector pCD188, BamHI restriction enzyme, and appropriate restriction buffer were used in the restriction endonuclease digestion step.

### B. BglII Digestion of Streptomycetes Vector pIJ940

Restriction endonuclease digestion of vector pIJ940 was performed essentially as described above except that about 2.5 µg of vector pIJ940, BglII restriction enzyme, and appropriate restriction buffer were used. Low copy number Streptomycetes vector pIJ940 was kindly supplied by the John Innes Institute, U.K.

### C. Ligation to Produce pCD425

About 0.6 µg of BamHI-linearized dephosphorylated pCD188, and about 2 µg of BglII-linearized pIJ940 were ligated at 15°C overnight (12-15 hours) as described above. The selected E. coli DH5-α transformant was designated as strain CD425. A restriction map of cosmid shuttle vector isolated from strain CD425, namely pCD425, is shown in FIG. 9.

### EXAMPLE 8

Transformation of Streptomyces lividans Protoplasts with Cosmid

### Shuttle Vector pCD425

S. lividans strain TK64 was used in this experiment. This strain has been very well analyzed and is widely used in streptomycetes research. The media for growing and protoplasting S. lividans, and the preparation of protoplasts and transformation were performed as described by D. A. Hopwood et al., Genetic Manipulation of Streptomyces - a laboratory manual, 1985, The John Innes Foundation, Norwich, U. K. This latter reference also describes the S. lividans strain TK64. S. lividans protoplasts transformed with shuttle vector pCD425 were selected for thiostrepton resistance. Vector pCD425 was found to transform S. lividans with a efficiency similar to its parent streptomycetes replicon, namely pIJ940.

While several examples of the present invention have been described, many changes and modifications may be made thereunto without departing from the spirit and scope of the invention.

## Claims

1. A cosmid cloning vector comprising:
(a) a PstI/ClaI DNA restriction fragment of about 4.2 kilobases, said restriction fragment comprising a ColEI origin of replication and at least one bacteriophage lambda cos site;
(b) a PstI/ClaI DNA fragment of about 0.8 kilobases, said DNA fragment comprising at least one BamHI cloning site flanked by at least two different bacteriophage promoter sequences and said BamHI cloning site further flanked by at least two restriction sites (SEQ ID NO:1); and
(c) a DNA segment containing at least one antibiotic marker that confers resistance when transformed into a sensitive E. coli host cell.

2. The cosmid cloning vector of claim 1, further comprising:
(d) an SspI linker (SEQ ID NO:2) inserted at the ClaI site; and
(e) at least three additional cloning sites designated as EcoRI, NotI and BamHI.

3. The cosmid cloning vector of claim 2 wherein the 4.2 kb PstI/ClaI DNA restriction fragment (SEQ ID NO:1) is derived from plasmid pCD184; the 0.8 kb PstI/ClaI DNA fragment is derived from pWE15, the BamHI cloning site is flanked by at least promoter sequences from bacteriophages T3 and T7 and the BamHI cloning site is further flanked by at least two restriction sites for enzymes NotI and EcoRI; and an SspI linker (SEQ ID NO:2) is inserted at the ClaI site described for plasmids pCD184 and pCD193 (SEQ ID NO:1).

4. The cosmid cloning vector of claim 1 wherein the cosmid cloning vector is a plasmid.

5. The plasmid of claim 4 werein the plasmid is selected from the group of plasmids designated as pCD188 (SEQ ID NO:1), pCD382 (SEQ ID NO:3), pCD383 (SEQ ID NO:3), pCD397 (SEQ ID NO:4), and pCD418 (SEQ ID NO:5).

6. An E. coli host cell comprising the cosmid cloning vector of claim 1.

7. The host cell of claim 6 wherein the cosmid cloning vector is a plasmid selected from the group of plasmids designated as pCD188 (SEQ ID NO:1), pCD382 (SEQ ID NO:3), pCD383 (SEQ ID NO:3), pCD397 (SEQ ID NO:4), and pCD418 (SEQ ID NO:5).

8. The cosmid cloning vector of claim 4 comprising all the structural characteristics of plasmid pCD188 and further comprising an additional XhoI cloning site located adjacent the BamHI cloning site.

9. An E. coli host cell comprising the cosmid cloning vector of claim 8.

10. The cosmid cloning vector of claim 4 comprising all the structural characteristics of plasmid pCD188 (SEQ ID NO:1) and further comprising additional XhoI and XbaI cloning sites located adjacent the BamHI cloning site (SEQ ID NO:3).

11. An E. coli host cell comprising the cosmid cloning vector of claim 10.

12. The cosmid cloning vector of claim 4 comprising all the structural characteristics of plasmid pCD188 and further comprising a multiple cloning site with the following configuration: EcoRI/NotI/BamHI/DraI/BglII/XhoI/ DraI/BamHI/NotI/EcoRI.

13. An E. coli host cell comprising the cosmid cloning vector of claim 12.

14. The cosmid cloning vector of claim 4 comprising all the structural characteristics present of plasmid pCD188 and further comprising a multiple cloning site with the following configuration: EcoRI/NotI/BamHI/AseI/DraI/BglII/ XhoI/DraI/AseI/BamHI/NotI/EcoRI.

15. An E. coli host cell comprising the cosmid cloning vector of claim 14.

16. A cosmid cloning vector comprising an E. coli origin of replication; at least one DNA segment that conveys resistance to an antibiotic when transformed into a sensitive E. coli host cell; at least one cos sequence from bacteriophage; at least one cloning site flanked by (Adenine+Thymine)-rich recognition sequences for restriction enzymes; and at least two different RNA polymerase promoters flanking the polylinker region.

17. A cosmid shuttle cloning vector that controls for a low copy number in streptomycetes cells, said cosmid shuttle cloning vector comprising:
(a) an E. coli origin of replication comprising a BamHI restriction fragment of about 5 kilobases;
(b) a DNA segment comprising at least one antibiotic marker that confers resistance when transformed into an E. coli host cell;
(c) a BglII restriction fragment of about 25 kilobases comprising a streptomycetes origin of replication;
(d) a DNA segment comprising at least one antibiotic marker that confers resistance when transformed into a sensitive Streptomyces host cell;
(e) a BamHI cloning site; and
(f) two ClaI sites flanking the BamHI cloning site.

18. The shuttle cloning vector of claim 17 wherein the BamHI restriction fragment is that of plasmid pCD188 (SEQ ID NO:1).

19. The shuttle cloning vector of claim 17 wherein the BglII restriction fragment is that of plasmid pIJ940.

20. The shuttle cloning vector of claim 17 wherein the vector is capable of replicating efficiently in a wide variety of streptomycetes and/or E. coli hosts.

21. The cosmid shuttle vector of claim 17 wherein the cosmid shuttle vector is a plasmid.

22. The plasmid of claim 21 designated pCD425.

23. A streptomycetes or E. coli host cell comprising the shuttle vector of claim 17.

24. The host cell of claim 23 selected from the group consisting of Streptomyces avermitilis; Streptomyces coelicolor; Streptomyces hygroscopicus; and Streptomyces lividans.

25. The host cell of claim 24 wherein the cloning shuttle vector is plasmid pCD425.

26. The host cell of claim 23 wherein the cloning shuttle vector is plasmid pCD425.
